# EUROPEAN PATENT SPECIFICATION

(11) **EP 3 174 565 B1**
(45) Date of publication and mention of the grant of the patent: **02.11.2022**
(21) Application number: 15753521.2
(22) Date of filing: 23.07.2015
(51) Int. Cl.: A61L 27/36, A01N 1/02, A61L 27/38, C12N 5/077

(54) **STORAGE MEDIA FOR TISSUE ALLOGRAFTS**
AUFBEWAHRUNGSMEDIEN FÜR GEWEBEALLOTRANSPLANTATE
MOYEN DE STOCKAGE POUR ALLOGREFFES DE TISSUS

(30) Priority: 30.07.2014 US 201462030897 P
(43) Date of publication of application: 07.06.2017
(73) Proprietor: Zimmer Orthobiologics, Inc., Austin, TX 78729-1103 (US)
(72) Inventor: MARGERRISON, Ed E. C., Austin, Texas 78733 (US); LIU, Hui, Austin, Texas 78727 (US); CLARKE, Rhonda, Cedar Park, Texas 78613 (US)
(74) Representative: Handley, Matthew Edward
(86) International application number: PCT/US2015/041717
(87) International publication number: WO 2016/018710

(56) References cited:
- WO-A1-91/07086
- KENJI ONUMA ET AL: "Allogenic Serum Improves Cold Preservation of Osteochondral Allografts", CLINICAL ORTHOPAEDICS AND RELATED RESEARCHÂ TM, SPRINGER-VERLAG, NEW YORK, vol. 470, no. 10, 18 November 2011 (2011-11-18), pages 2905-2914, XP035111896, ISSN: 1528-1132, DOI: 10.1007/S11999-011-2182-6

## Description

### CLAIM OF PRIORITY

This application claims priority from U.S. Provisional Patent Application Serial No. 62/030,897, filed on July 30, 2014.

### TECHNICAL FIELD

This document pertains to methods of producing allografts for allotransplantation having an extended shelf life.

### BACKGROUND

Allotransplantation is the transplantation of allografts of donor tissue into a prepared site in corresponding tissue in the recipient to repair damaged tissue or supplement existing healthy tissue. Allografts typically comprise frozen tissue or "fresh", unfrozen tissue in a storage media. While freezing the tissue allows the tissue to be safely stored for extended periods of time before being thawed and implanted, the freezing process kills the live cells in the tissue. In contrast, fresh tissue allografts contain live cells that can improve the likelihood that the allograft will successfully integrate with the surrounding tissue. However, the live cells in the fresh tissue allografts will naturally degrade overtime reducing the benefits provided by the live tissue allograft. The time period for implanting certain types of fresh tissue allografts with sufficient numbers of live cells to improved functionality and durability can be as short as one week. As allografts are produced from donor tissue, the limited supply and shelf life of live tissue allografts substantially weigh against the use of fresh tissue grafts despite the substantial benefit of improved functionality and durability. Methods of maintaining cell viability are known, for example, from WO 91/07086, and methods of maintaining the viability of chondrocytes in particular are known from Onuma et al., "Allogenic Serum Improves Cold Preservation of Osteochondral Allografts", Clinical Orthopaedics And Related Research, vol. 470, no. 10, pages 2905 - 2914.

Osteochondral allotransplantation is a common form of allotransplantation in which allografts containing cartilage and bone are transplanted to a recipient to repair or replace damaged cartilage. In particular, osteochondral allotransplantation is often used to repair lesions in the cartilage covering the condyle and cushioning the interface between the femur, tibia or patella. Each allograft for osteochondral allotransplantation typically includes a cartilage portion containing chondrocyte cells for repairing the cartilage and a bone portion for positioning and fixing the cartilage portion. An osteochondral allotransplantation procedure typically involves drilling or punching a hole through the lesion in the cartilage and into the underlying condyle bone to excise the lesion and a portion of the bone. The removal of the bone section provides a rigid mounting point for the allograft and can induce bleeding to promote in-growth of the surrounding bone into the bone portion of the allograft to fuse the allograft to bone. The allograft is shaped and sized to approximate the shape and depth of the bored out portion of the bone and cartilage before being inserted into the hole. During a successful allotransplantation, the surrounding healthy cartilage tissue will integrate with the cartilage tissue of the allograft.

Cartilage portions of osteochondral allografts can be particularly difficult to integrate with the surrounding tissue. Similarly, donor tissue for producing osteochondral allografts can be more limited than other tissue. Accordingly, the inability to effectively supply fresh tissue osteochondral allografts is particularly challenging.

### OVERVIEW

The present invention is defined by the appended claims, any subject matter falling outside of the claimed subject matter is disclosed for information only.

The present inventors have recognized, among other things, that a problem to be solved can include the high rate of cell death in live tissue allografts. The present subject matter can provide a solution to this problem, such as by applying a storage media containing at least one free-radical scavenger to the live tissue allograft. The free-radical scavenger comprises N-Acetylcysteine and it can remove reactive elements in the allograft tissue and shield the allograft tissue from additional free radicals from entering the tissue during storage. The removed reactive elements and free radicals can reduce the rate of cell death, allowing implanting of the allograft tissue with a high level of live cells to improve functionality and durability of the allograft. The storage media can also include at least one additive for facilitating normal cellular activity to further reduce the rate of cell death. The present invention is defined in the independent claim, to which reference should now be made. Advantageous embodiments are set out in the sub claims.

An allograft having an extended shelf life can be prepared by a process comprising:excising a quantity of allograft tissue comprising a quantity of live chondrocytes from a donor source;maintaining the excised allograft tissue above a predetermined temperature; andapplying a storage media to the excised allograft tissue, the storage media containing at least one free radical scavenger for reducing free-radicals in the excised allograft tissue, wherein the free radical scavenger comprises N-Acetylcysteine.

The storage media and storage, can maintain viability of cells contained within the excised tissue such that at least 95 % of the cells are viable after one week. Similarly, the storage media and storage, according to an example of the present subject matter, can maintain viability of cells contained within the excised tissue such that at least 70 % of the cells are viable after four weeks.

In an example, a method of transplanting a live tissue allograft (not part of the present invention) can include providing allograft tissue from a donor source. The method can further include maintaining the allograft tissue above a predetermined temperature and applying a storage media to the allograft tissue containing at least one free radical scavenger for reducing free radicals in the storage media and the allograft tissue. The method can also include excising damaged tissue from recipient tissue to form a recipient site and inserting the allograft tissue into the recipient site.

In an example, a fresh tissue osteochondral allograft (not part of the invention) can include a cartilage portion and a bone portion. The osteochondral allograft can also include a storage media containing at least one free radical scavenger for reducing free-radicals.

This overview is intended to provide an overview of subject matter of the present patent application. It is not intended to provide an exclusive or exhaustive explanation of the present subject matter. The detailed description is included to provide further information about the present patent application.

### BRIEF DESCRIPTION OF THE DRAWINGS

In the drawings, which are not necessarily drawn to scale, like numerals may describe similar components in different views. Like numerals having different letter suffixes may represent different instances of similar components. The drawings illustrate generally, by way of example, but not by way of limitation, various embodiments discussed in the present document.
Figure 1 is a perspective view of an allograft having allograft tissue according to an example of the present subject matter.
Figure 2 is a schematic diagram of the preparation of an allograft according to an example of the present subject matter.
Figure 3 is a schematic diagram of the preparation of an allograft according to an example of the present subject matter.
Figure 4 is a representative plot of cell viability over an extended storage duration of chondrocytes prepared according to various examples of the present subject matter as compared to chondrocytes stored in a conventional storage media.
Figure 5 is a representative plot of cell viability over an extended storage duration of chondrocytes prepared according to various examples of the present subject matter as compared to chondrocytes stored in a conventional storage media.
Figure 6 is a representative plot of cell viability an over extended storage duration of chondrocytes prepared according to various examples of the present subject matter as compared to chondrocytes stored in a conventional storage media.
Figure 7 is a representative plot of cell viability over an extended storage duration of chondrocytes prepared according to various examples of the present subject matter containing different combinations of additives as compared to chondrocytes stored in a conventional storage media.
Figure 8 is a representative plot of cell viability over an extended storage duration of chondrocytes prepared according to various examples of the present subject matter containing different combinations of additives as compared to chondrocytes stored in a conventional storage media.
Figure 9 is a representative plot of cell viability over an extended storage duration of chondrocytes prepared according to various examples of the present subject matter containing different combinations of additives as compared to chondrocytes stored in a conventional storage media.
Figure 10 is a representative plot of cell viability over an extended storage duration of cell viability over an extended storage duration of chondrocytes prepared according to an example of the present subject matter as compared to chondrocytes stored in a conventional storage media.
Figure 11 is a representative plot of a ratio of live cells after an extended storage duration to live cells at the start of storage of chondrocytes prepared according to an example of the present subject matter as compared to chondrocytes stored in a conventional storage media.
Figure 12 is a representative plot of a ratio of live cells after an extended storage duration to live cells at the start of storage of chondrocytes prepared according to an example of the present subject matter as compared to an chondrocytes stored in a conventional storage media.
Figure 13 is a representative plot of a ratio of live cells after an extended storage duration to live cells at the start of storage of chondrocytes prepared according to an example of the present subject matter as compared to chondrocytes stored in a conventional storage media.

### DETAILED DESCRIPTION

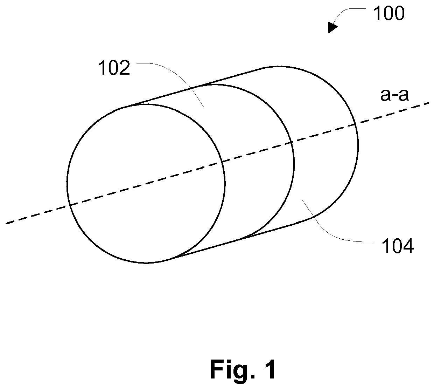

As depicted in FIG. 1, live allograft tissue 100 for allotransplantation and having an extended viable life, according to an example, includes a first tissue portion 102 and an applied storage media. The storage media reduces the death rate of live cells in at least the first tissue portion 102. A sufficiently large number of live cells in the first tissue portion 102 at implantation can contribute to improved functionality and durability of the allograft tissue 100 with the surrounding tissue at the recipient site for the allograft tissue 100. Certain types of allograft tissue 100 contain cells that, when implanted live, can continue to live in the recipient's body following transplantation, which can improved functionality and durability. Reducing the death rate of live cells in the first tissue portion 102 can increase the time period for which the allograft tissue 100 can be transplanted with improved functionality and durability provided by the live cells in the first tissue portion 102.

In an example, the first tissue portion 102 can comprise, but is not limited to, bone, bone marrow, cartilage, corneas, ligaments, intestines, whole or partial organs, skin, tendons or other tissues in which a quantity of the cells remain viable at implantation. In certain examples, allograft tissue 100 can comprise a second tissue portion 104, wherein the tissue of the second tissue portion 104 corresponds to the tissue of the first tissue portion 102. In an example, the second tissue portion 104 can comprise tissue that is ordinarily adjacent to the tissue of the first tissue portion 102. The second tissue portion 104 can be used to position or anchor the first tissue portion 102 during integration of the first tissue portion 102 into the tissue surrounding the recipient cite. In an example, the allograft tissue 100 can comprise osteochondral tissue, wherein the first tissue portion 102 comprises cartilage tissue and the second tissue portion 104 comprises bone. In this configuration, the first tissue portion 102 contains chondrocytes that integrate with cartilage tissue surrounding the recipient site.

In an example, the storage media comprises a free radical scavenger, the free radical scavenger comprises N-Acetylcysteine, for reducing free radicals in at least the first tissue portion 102. Removing free radicals in the first tissue portion 102 can prevent undesirable reactions caused by the first tissue portion 102 that can result in an increased rate of cell death of live cells in the first tissue portion 102. Cell growth of new cells in the first tissue portion 102 effectively ceases after the first tissue portion 102 is excised from the donor tissue, which removes the live cells from the nutritional sources. The lack of nutritional sources also causes the live cells to die. Certain cell types, such as immunoprivileged chondrocytes, are capable of surviving for extended time periods following excising from the donor tissue, but can be prematurely damaged or die from reactions with free radicals. In an example, the free radical scavenger can comprise an oxygen radical scavenger or antioxidant for removing oxygen radicals from at least the first tissue portion 102. The oxygen radicals can cause oxidative stress in the live cells resulting in damage to the live cells and ultimately cell death. Scavenging the oxygen radicals can remove a substantial cause of cell death reducing the rate of cell death and extending the time period in which the first tissue portion 102 can be implanted into the recipient site with sufficient live cells to improve functionality and durability of the first tissue portion 102. In an example, the oxygen radical scavenger or antioxidant can include, but is not limited to, Vitamin C (ascorbic acid), Vitamin E (tocotrienols, tocopherols,), Carotenoids (carotenes, lycopene, lutein), Polyphenolic antioxidants (resveratrol, flavonoids), Glutathione, Selenium and N-Acetylcysteine.

In an example, the storage media can comprise at least one additive for maintaining proper cellular function of live cells following excising for the first tissue portion 102. In certain examples, the additive can comprise a base medium or other energy source to support cellular metabolic activity or growth. The base medium or other energy source can include, but is not limited to, L-Alanyl-Glutamine, Dulbecco's modified Eagle's medium ("DMEM"), linoleic acid, sodium pyruvate and combinations thereof. In certain examples, the additive can prevent formation of undesirable byproducts, such as ammonia byproducts, that result from excising of the first tissue portion 102 or are ordinarily removed from the tissue by other processes. These additives can include, but are not limited to, L-Alanyl-L-Glutamine, transferrin, dexamethasone, N-Acetylcystenine and Glutathione. In certain examples, the additive can prevent programmed cell death ("PCD") and can comprise, but is not limited to, resveratrol. In certain examples, the additive can supply nutrients and other chemicals for normal cellular function such as, but not limited to, amino acid and glucose uptake, cellular structure construction, intracellular transport, lipogenesis, nucleic acid and protein synthesis, and pH regulation. These additives can include, but are not limited to, hyaluronic acid, N-2-hydroxyelthlpiperazine-N'-2-Ethanesulfonic acid ("HEPES"), insulin, insulin-like growth factors, linoleic acid, selenium, and transferrin.

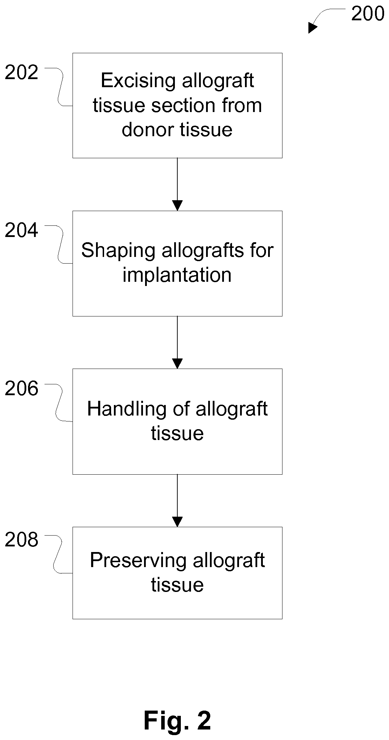

As depicted in FIG. 2, a method 200 of preparing allograft tissue 100 having an extended viable life, according to an example, can comprise excising 202, shaping 204, handling 206 and preservation 208.

At 202, a portion of donor tissue is excised to form a section of allograft tissue 100 containing at least one allograft. In an example, each allograft can be individually excised from the donor tissue. In another example, a segment of allograft tissue 100 can be excised from the donor tissue and subdivided into individual allografts. In this configuration, the segment of allograft tissue 100 can be divided such that each allograft includes tissue of the first tissue portion 102 and tissue of the second tissue portion 104 such as depicted in FIG. 1.

At 204, the allografts are shaped for efficient implantation. In an example, each allograft can be formed into a cylindrical shape as depicted in FIG. 1 such that the allograft can be inserted into a cylindrical bore hole formed in the recipient site. The cylindrical shape can simplify the formation of the recipient site and conforming the shape and size of the allograft tissue 100 with the recipient site. In this configuration, the allograft tissue 100 can include a standard diameter, wherein the length of the allograft tissue 100 can be varied to fit the depth of the recipient site. In an example, each allograft of allograft tissue 100 can include at least a first tissue portion 102 and a second tissue portion 104, each allograft portion being shaped such that the first tissue portion 102 and the second tissue portion 104 align with the corresponding tissue upon insertion into the recipient site. In certain examples, each allograft can be shaped to correspond to the shape of the intended recipient site.

At 206, ,each allograft is maintained above a predetermined temperature or range of temperatures, wherein the predetermined temperature corresponds to the freezing temperature of the allograft tissue 100. In an example, the tissue can be maintained at a temperature greater than 0 °C. In another example, the tissue can be maintained at a temperature between about 0 °C to about 370 °C. In yet another example, the tissue can be maintained at a temperature between about 19 °C to 26 °C. While freezing the allograft tissue 100 will preserve the allograft tissue 100, the freezing of the tissue can result in the death of live cells in at least the first tissue portion 102. In an example, the first tissue portion 102 and the second tissue portion 104 can comprise different freezing temperatures, wherein the allograft is maintained above the greater freezing temperature.

At 208, storage media containing at least one free radical scavenger comprising N-Acetylcysteine is applied to each allograft portion. The storage media can be applied by a topical spray, liquid coating, submersion or other approaches for applying a liquid or semisolid material to each allograft portion. In an example, the storage media can permeate the allograft to remove free radicals throughout the allograft.

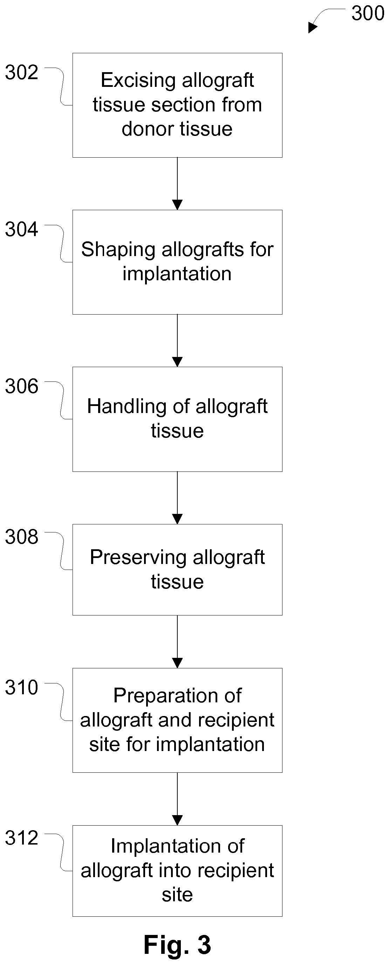

As depicted in FIG. 3, a method 300 of transplanting a live tissue allograft (method not part of the present invention) having an extended viable life, according to an example, can comprise excising 302, shaping 304, handling 306, preservation 308, preparation 310 and placement 312.

At 302, a portion of donor tissue can be excised to form a section of allograft tissue 100 containing at least one allograft. In an example, each allograft can be individually excised from the donor tissue. In another example, a segment of allograft tissue 100 can be excised from the donor tissue and subdivided into individual allografts. In this configuration, the segment of allograft tissue 100 can be divided such that each allograft includes tissue of the first tissue portion 102 and tissue of the second tissue portion 104 such as depicted in FIG. 1. In this configuration, the relative proportions of the first tissue portion 102 and second tissue portion 104 can correspond to the tissue proportions at the recipient site.

At 304, the allograft is shaped into a predetermined shape corresponding to the desired recipient site. In certain examples, the recipient site can be formed as a bore hole comprising a standard shape, such as a cylindrical bore hole. In this configuration, each allograft can be formed into a cylindrical shape as depicted in FIG. 1 such that the allograft can be inserted into the cylindrical bore hole formed in the recipient site. The allograft can comprise a pre-determined diameter corresponding to a standard size for the bore hole at the recipient site. In this configuration, the allograft can be trimmed along axis a-a to adjust the length of the allograft to correspond to the depth of the bore hole. The corresponding diameter of the allograft and the bore hole can simplify and shorten the surgical implantation. In an example, each allograft of allograft tissue 100 can include at least a first tissue portion 102 and a second tissue portion 104, each allograft portion being shaped such that the first tissue portion 102 and the second tissue portion 104 align with the corresponding tissue upon insertion into the recipient site.

At 306, in an example, each allograft can be maintained above a predetermined temperature or range of temperatures, wherein the predetermined temperature corresponds to the freezing temperature of the allograft tissue 100. While freezing the allograft tissue 100 will preserve the allograft tissue 100, the freezing of the tissue can result in the death of live cells in the first tissue portion 102. In an example, the first tissue portion 102 and the second tissue portion 104 can comprise different freezing temperatures, wherein the allograft is maintained above the greater freezing temperature.

At 308, storage media containing at least one free radical scavenger, comprising N-Acetylcysteine, can be applied to each allograft portion. The storage media can be applied by a topical spray, liquid coating, submersion or other approaches for applying a liquid or semisolid material to each allograft portion. In an example, the storage media can permeate the allograft to remove free radicals throughout the allograft.

At 310, a recipient site can be prepared to receive the allograft. In an example, a bore hole can be cut into the damaged tissue or lesion to form the recipient site. In certain examples, the recipient site can comprise a cylindrical shaped bore hole surrounded by tissue corresponding to at least the first tissue portion 102. In an example, the bore hole can be cut to sufficient depth to expose an underlying tissue layer, wherein the underlying tissue layer corresponds to the second tissue portion 104. In an example, the length of the allograft can be trimmed to correspond to the depth of the bore hole.

At 312, in an example, the allograft can then be inserted into the recipient site such that a face of the allograft generally aligns with the tissue adjacent the recipient site. In an example, the allograft can be inserted such that the tissue of the first tissue portion 102 and the second tissue portion 104 align with corresponding tissue adjacent to the recipient site.

Each of these non-limiting examples can stand on its own, or can be combined in any permutation or combination with any one or more of the other examples.

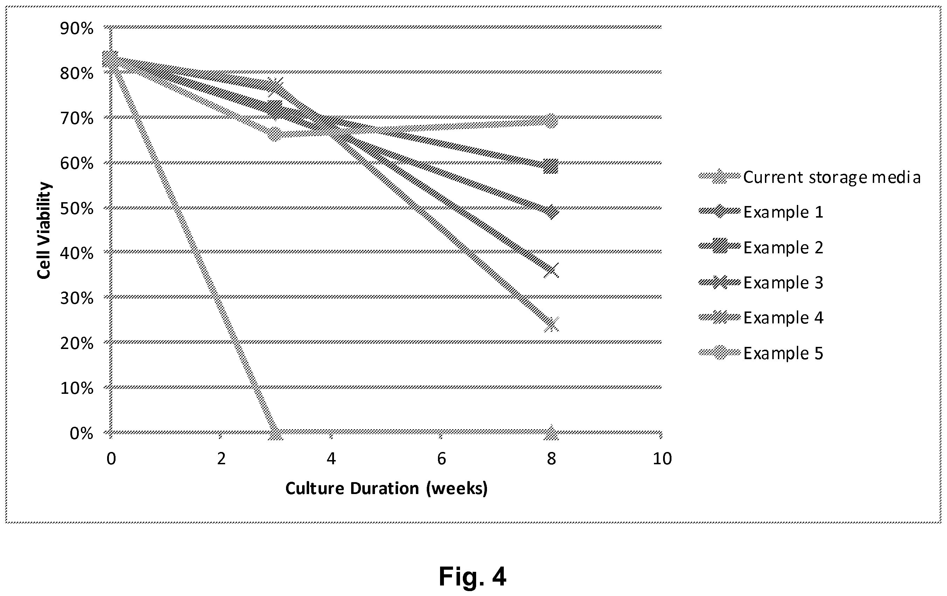

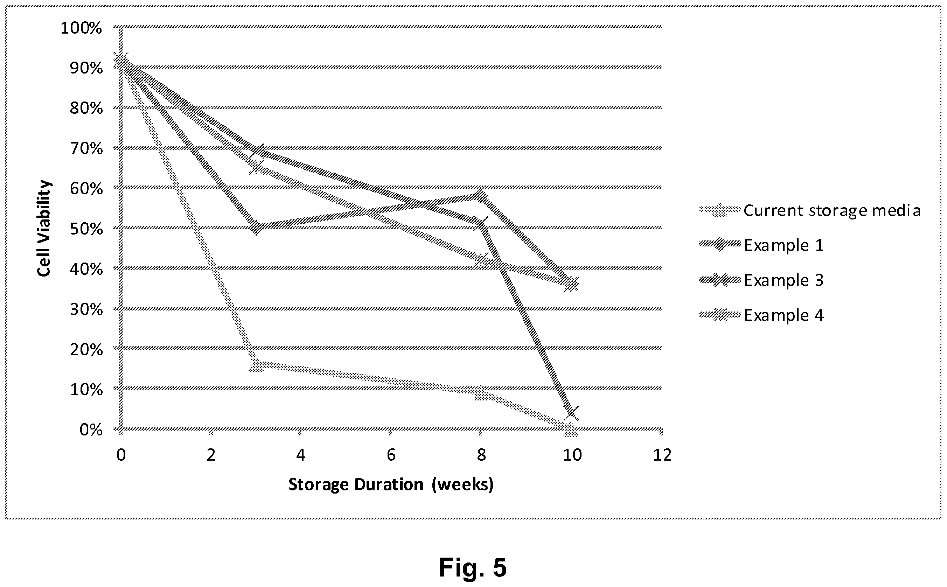

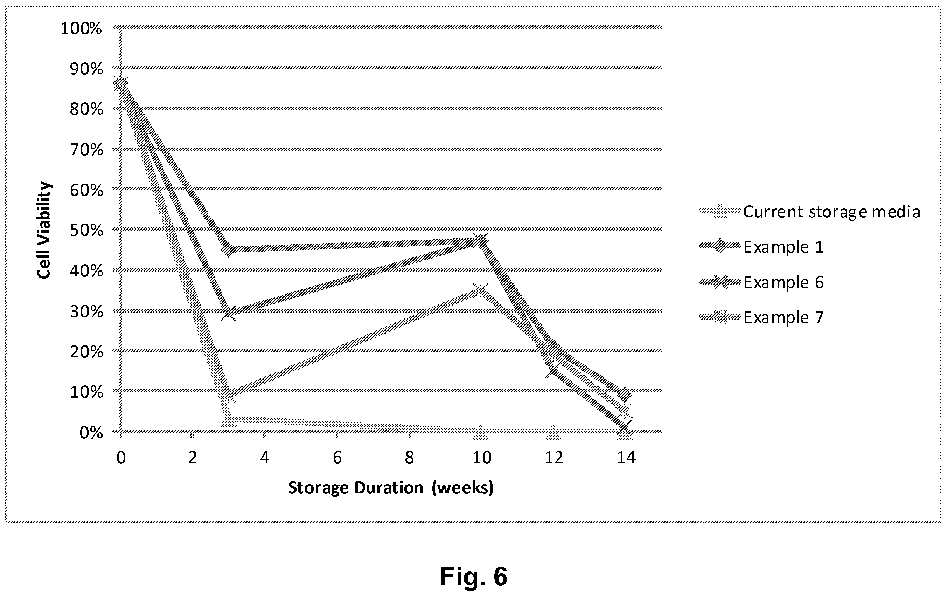

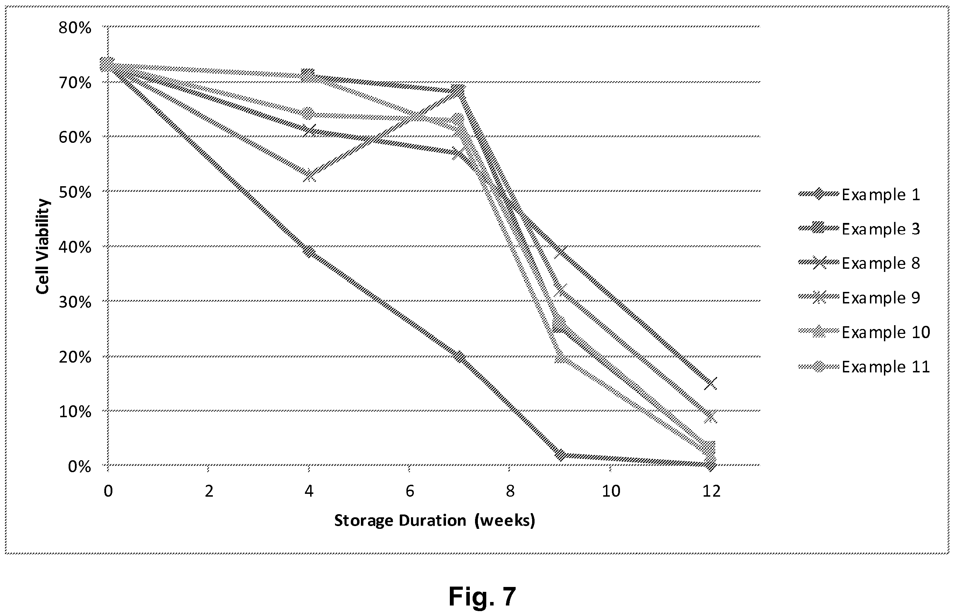

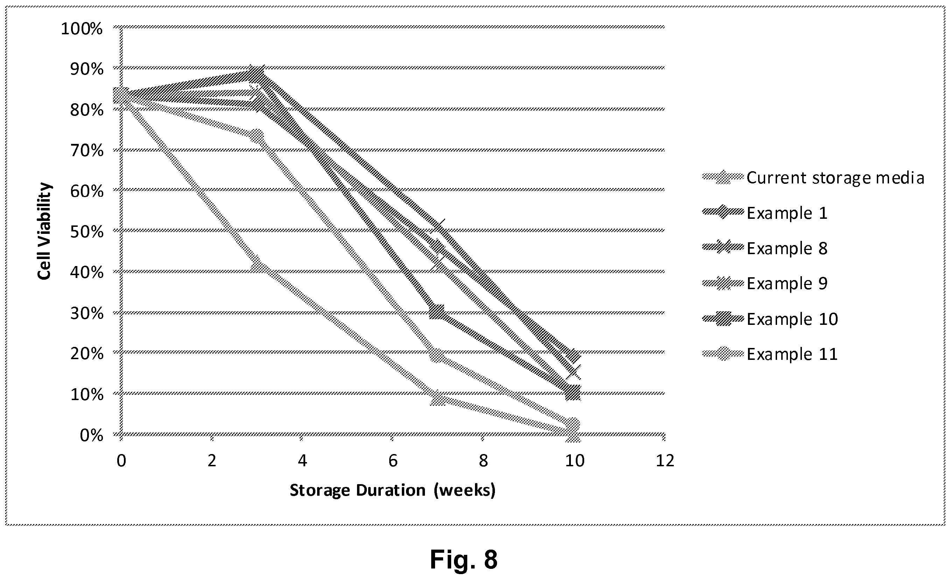

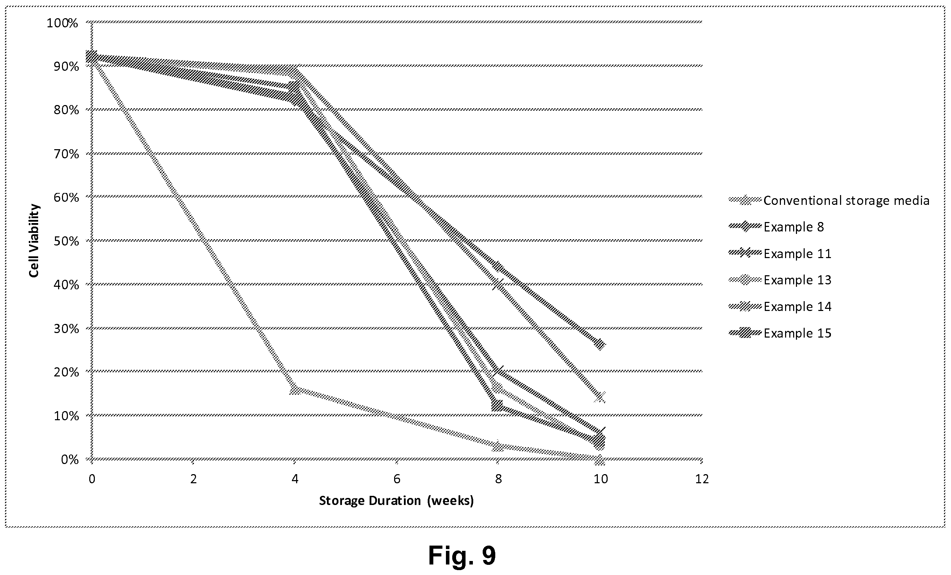

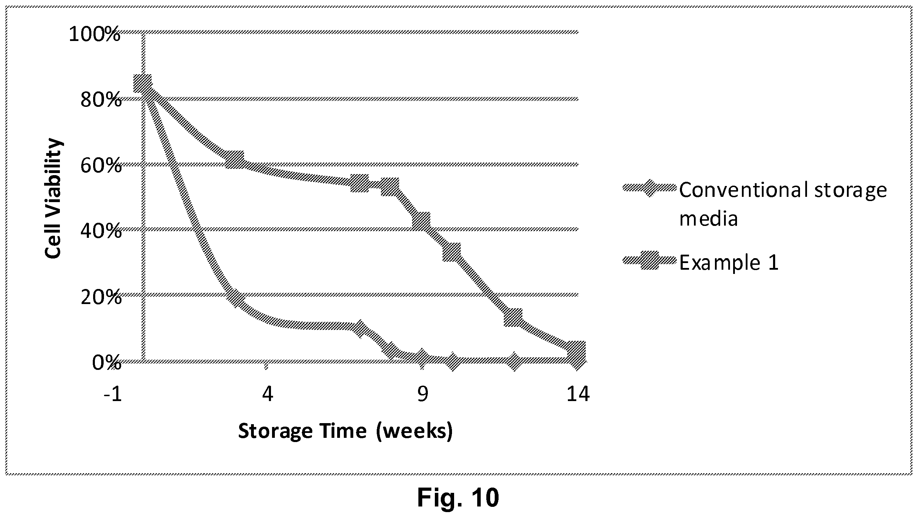

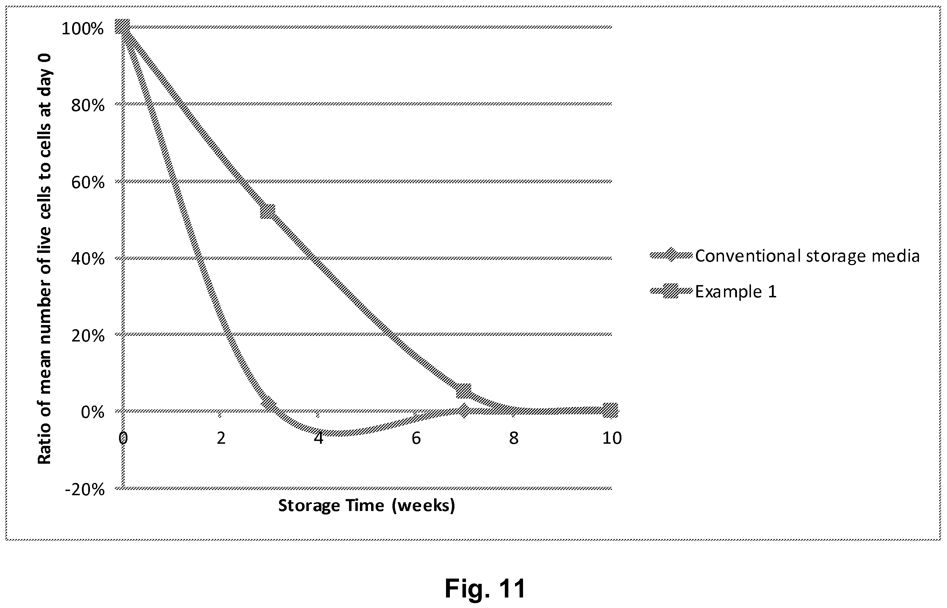

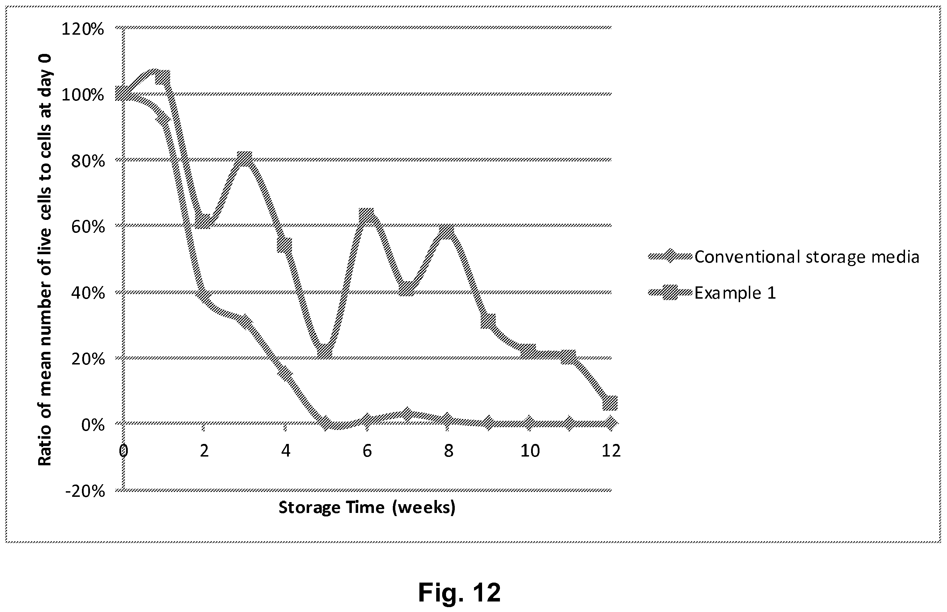

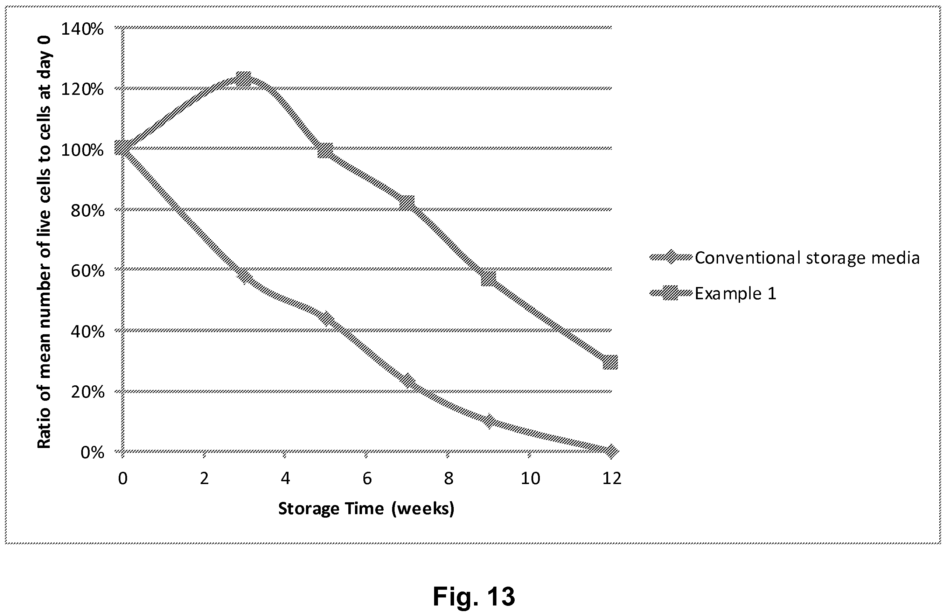

FIGS. 4-10 are representative plots of cell viability over an extended storage duration of chondrocytes prepared according to various Examples of the present subject matter as compared to chondrocytes stored in a conventional storage media. A conventional storage media as referenced in the present disclosure can include amino acids, vitamins, inorganic salts, carbohydrates and serum. As illustrated in FIGS. 4-10, the rate of cell death in a conventional storage media is about 10% of the total live cells per week. A storage media according to an example of the present disclosure can reduce cell death to at least about 5 % of the total live cells per week. FIGS. 11-13 are representative plots of a ratio of live cells after an extended storage duration to live cells at the start of storage of an cartilage allograft prepared according to various Examples of the present subject matter as compared to an allograft stored in a conventional storage media. The various Examples depicted in FIGS. 4-13 are described in further detail below.

Example 1 can include allograft tissue to which a storage media including at least one free radical scavenger is applied. In an example, the at least one free radical can include an oxygen free-radical scavenger such as N-Acetyl-L-Cysteine.

Example 2 can include the formulation of Example 1 and further include at least one antibiotic. In an example, the antibiotic can comprise penicillin streptomycin.

Example 3 can include the formulation of Examples 1 or 2 and further include a serum-free serum replacement for growing and maintaining cells in culture. In an example, the serum replacement can comprise KNOCK OUT SERUM REPLACEMENT produced by LIFE TECHNOLOGIES of Carlsbad, CA.

Example 4 can include the formulation of Example 3 and further include a second free-radical scavenger. In an example, the second free-radical scavenger can comprise methylene blue.

Example 5 can include the formulation of any one of the proceeding Examples and further include a second free-radical scavenger. In an example, the second free-radical scavenger can comprise methylene blue.

Example 6 can include the formulation of any one of the proceeding Examples, wherein the at least one free-radical scavenger can comprise a reduced concentration.

Example 7 can include the formulation of any one of the proceeding Examples and further include at least one anti-apoptotic agent. In an example, the at least one apoptotic agent can comprise hyaluronic acid.

Example 8 can include the formulation of any one of the proceeding Examples and further include at least a second free radical scavenger. In an example, the second free radical scavenger can comprise vitamin E.

Example 9 can include the formulation of Example 8 and further include at least one anti-apoptotic agent. In an example, the at least one anti-apoptotic agent can comprise insulin-like growth factor.

Example 10 can include the formulation of any one of proceeding Examples 9-10 and further include a serum-free serum replacement. In an example, the serum replacement can include KNOCK OUT SERUM REPLACEMENT.

Example 11 can include the formulation of any one of proceeding Examples 8-10 and further include a serum-free serum replacement. In an example, the serum replacement can include KNOCK OUT SERUM REPLACEMENT.

Example 12 can include the formulation of any one of the proceeding Examples, wherein the concentration of the at least one free-radical scavenger is doubled. In an example, the at least one free radical can include an oxygen free-radical scavenger such as N-Acetyl-L-Cysteine.

Example 13 can include the formulation of Example 12 and further include a serum replacement and a second free radical scavenger. In an example, the serum replacement can comprise KNOCK OUT SERUM REPLACEMENT and the second free-radical scavenger can comprise vitamin E.

Example 14 can include the formulation of any one of proceeding Examples 8-13 and further include a third free-radical scavenger. In an example, third free-radical scavenger can comprise Resveratrol 20.

Example 15 can include the formulation of any one of proceeding Examples 8-14 and further include a third free-radical scavenger. In an example, third free-radical scavenger can comprise Resveratrol 100.

As depicted in FIGS. 4-10, allografts having a storage media according to an example of the present subject matter can have improved cell viability over time as compared to conventional storage media. As depicted in FIGS. 11-13, allografts having a storage media according to an example of the present subject matter can have a higher ratio of live cells after an extended duration to live cells at the initial live cell count than allografts having conventional storage media.

Each of these non-limiting Examples can stand on its own, or can be combined in any permutation or combination with any one or more of the other Examples.

The above detailed description includes references to the accompanying drawings, which form a part of the detailed description. The drawings show, by way of illustration, specific embodiments in which the present subject matter can be practiced. These embodiments are also referred to herein as "examples." Such examples can include elements in addition to those shown or described. However, the present inventors also contemplate examples in which only those elements shown or described are provided. Moreover, the present inventors also contemplate examples using any combination or permutation of those elements shown or described (or one or more aspects thereof), either with respect to a particular example (or one or more aspects thereof), or with respect to other examples (or one or more aspects thereof) shown or described herein.

In this document, the terms "a" or "an" are used, as is common in patent documents, to include one or more than one, independent of any other instances or usages of "at least one" or "one or more." In this document, the term "or" is used to refer to a nonexclusive or, such that "A or B" includes "A but not B," "B but not A," and "A and B," unless otherwise indicated. In this document, the terms "including" and "in which" are used as the plain-English equivalents of the respective terms "comprising" and "wherein." Also, in the following claims, the terms "including" and "comprising" are open-ended, that is, a system, device, article, composition, formulation, or process that includes elements in addition to those listed after such a term in a claim are still deemed to fall within the scope of that claim. Moreover, in the following claims, the terms "first," "second," and "third," etc. are used merely as labels, and are not intended to impose numerical requirements on their objects. the claims. Also, in the above Detailed Description, various features may be grouped together to streamline the disclosure.

## Claims

1. An allograft having an extended shelf life prepared by a process comprising:
excising a quantity of allograft tissue comprising a quantity of live chondrocytes from a donor source;
maintaining the excised allograft tissue above a predetermined temperature; and
applying a storage media to the excised allograft tissue, the storage media containing at least one free radical scavenger for reducing free-radicals in the excised allograft tissue, wherein the free radical scavenger comprises N-Acetylcysteine.

2. The allograft of claim 1, wherein the excised allograft tissue comprises a first tissue portion and a second tissue portion.

3. The allograft of claim 1 or 2, wherein the excised allograft tissue contains a cartilage tissue portion comprising the quantity of live chondrocytes.

4. The allograft of claim 3, wherein the excised allograft tissue comprises osteochondral tissue, wherein the first tissue portion comprises a bone tissue portion and the second tissue portion comprises the cartilage tissue portion.

5. The allograft of claim 3, wherein the storage media further comprises vitamin E, resveratrol, hyaluronic acid, or methylene blue.

6. The allograft of any one of the preceding claims,
wherein at least about 95% of the quantity of live cells are viable after maintaining the allograft tissue in the storage media for a week.

7. The allograft of any one of the preceding claims
wherein at least about 70% of the quantity of live cells are viable after 4 weeks.

8. The allograft of any one of the preceding claims, the process further comprising:
shaping the excised allograft tissue into a cylindrical shape.

9. The allograft of any one of the preceding claims, wherein the predetermined temperature is 19-26°C.

10. The allograft of any one of the preceding claims, wherein the storage media further comprises a serum-free serum replacement, or an anti-apoptotic agent.

11. The allograft of claim 4, for use in a method of allotransplantation, the method comprising:
excising damaged tissue from recipient tissue to form a recipient site in the recipient tissue; and
inserting the excised allograft tissue into the recipient site.

12. An allograft for use as claimed in claim 11, wherein the method further comprises:
aligning the bone portion and the cartilage portion of the allograft tissue with corresponding tissue at the recipient site.

13. An allograft for use as claimed in any one of claims 11 or 12, wherein the recipient site comprises a cylindrical bore hole, and wherein the method further comprises shaping the excised allograft tissue into a cylindrical shape.

14. An allograft for use as claimed in any one of claims 12-14, wherein the method further comprises:
measuring a depth of the recipient site; and
cutting the allograft tissue to a length corresponding to the measured depth of the recipient site.

## Patentansprüche

1. Allotransplantat, das eine verlängerte Haltbarkeit aufweist, hergestellt durch ein Verfahren, umfassend:
Exzidieren einer Menge von Allotransplantatgewebe, umfassend eine Menge lebender Chondrozyten aus einer Donorquelle;
Halten des exzidierten Allotransplantatgewebe über einer vorbestimmten Temperatur; und
Anwenden eines Lagermediums auf das exzidierte Allotransplantatgewebe, wobei das Lagermedium mindestens einen Radikalfänger zur Reduktion freier Radikale in dem exzidierten Allotransplantatgewebe enthält, wobei der Radikalfänger N-Acetylcystein umfasst.

2. Allotransplantat nach Anspruch 1, wobei das exzidierte Allotransplantatgewebe einen ersten Gewebeabschnitt und einen zweiten Gewebeabschnitt umfasst.

3. Allotransplantat nach Anspruch 1 oder 2, wobei das exzidierte Allotransplantatgewebe einen Knorpelgewebeabschnitt enthält, umfassend die Menge an lebenden Chondrozyten.

4. Allotransplantat nach Anspruch 3, wobei das exzidierte Allotransplantatgewebe osteochondrales Gewebe umfasst, wobei der erste Gewebeabschnitt einen Knochengewebeabschnitt umfasst und der zweite Gewebeabschnitt den Knorpelgewebeabschnitt umfasst.

5. Allotransplantat nach Anspruch 3, wobei das Lagermedium ferner Vitamin E, Resveratrol, Hyaluronsäure oder Methylenblau umfasst.

6. Allotransplantat nach einem der vorherigen Ansprüche,
wobei mindestens etwa 95 % der Menge an lebenden Zellen lebensfähig sind, nachdem das Allotransplantatgewebe eine Woche lang in dem Lagermedium aufbewahrt wurde.

7. Allotransplantat nach einem der vorherigen Ansprüche
wobei mindestens etwa 70 % der Menge an lebenden Zellen nach 4 Wochen lebensfähig sind.

8. Allotransplantat nach einem der vorherigen Ansprüche, wobei das Verfahren ferner Folgendes umfasst: Formen des exzidierten Allotransplantatgewebes in eine zylindrische Form.

9. Allotransplantat nach einem der vorherigen Ansprüche, wobei die vorbestimmte Temperatur 19-26 °C ist.

10. Allotransplantat nach einem der vorherigen Ansprüche, wobei das Lagermedium ferner einen serumfreien Serumersatz oder ein anti-apoptotisches Mittel umfasst.

11. Allotransplantat nach Anspruch 4 zur Verwendung in einem Allotransplantationsverfahren, wobei das Verfahren Folgendes umfasst:
Exzidieren von geschädigtem Gewebe aus dem Empfängergewebe, um eine Empfängerstelle in dem Empfängergewebe zu bilden; und
Einsetzen des exzidierten Allotransplantats in die Empfängerstelle.

12. Allotransplantat zur Verwendung nach Anspruch 11, wobei das Verfahren ferner Folgendes umfasst:
Ausrichten des Knochenabschnitts und des Knorpelabschnitts des Allotransplantats mit dem entsprechenden Gewebe an der Empfängerstelle.

13. Allotransplantat zur Verwendung nach einem der Ansprüche 11 oder 12, wobei die Empfängerstelle ein zylindrisches Bohrloch umfasst und wobei das Verfahren ferner ein Formen des exzidierten Allotransplantatgewebes in eine zylindrische Form umfasst.

14. Allotransplantat zur Verwendung nach einem der Ansprüche 12-14, wobei das Verfahren ferner Folgendes umfasst:
Messen einer Tiefe der Empfängerstelle; und
Zuschneiden des Allotransplantats auf eine Länge, die der gemessenen Tiefe der Empfängerstelle entspricht.

## Revendications

1. Allogreffe ayant une durée de conservation prolongée préparée par un processus comprenant :
l'excision d'une quantité de tissu d'allogreffe comprenant une quantité de chondrocytes vivants provenant d'une source donneuse ;
le maintien du tissu d'allogreffe excisé au-dessus d'une température prédéterminée ; et
l'application d'un milieu de stockage au tissu d'allogreffe excisé, le milieu de stockage contenant au moins un piégeur de radicaux libres pour réduire les radicaux libres dans le tissu d'allogreffe excisé, dans lequel le piégeur de radicaux libres comprend de la N-acétylcystéine.

2. Allogreffe selon la revendication 1, dans laquelle le tissu d'allogreffe excisé comprend une première partie de tissu et une seconde partie de tissu.

3. Allogreffe selon la revendication 1 ou 2, dans laquelle le tissu d'allogreffe excisé contient une partie de tissu cartilagineux comprenant la quantité de chondrocytes vivants.

4. Allogreffe selon la revendication 3, dans laquelle le tissu d'allogreffe excisé comprend du tissu ostéochondral, dans laquelle la première partie de tissu comprend une partie de tissu osseux et la seconde partie de tissu comprend la partie de tissu cartilagineux.

5. Allogreffe selon la revendication 3, dans laquelle le milieu de stockage comprend en outre de la vitamine E, du resvératrol, de l'acide hyaluronique ou du bleu de méthylène.

6. Allogreffe selon l'une quelconque des revendications précédentes,
dans laquelle au moins environ 95 % de la quantité de cellules vivantes sont viables après maintien du tissu d'allogreffe dans le milieu de stockage pendant une semaine.

7. Allogreffe selon l'une quelconque des revendications précédentes,
dans laquelle au moins environ 70 % de la quantité de cellules vivantes sont viables après 4 semaines.

8. Allogreffe selon l'une quelconque des revendications précédentes, le processus comprenant en outre :
la mise en forme du tissu d'allogreffe excisé en une forme cylindrique.

9. Allogreffe selon l'une quelconque des revendications précédentes, dans laquelle la température prédéterminée est de 19 à 26°C.

10. Allogreffe selon l'une quelconque des revendications précédentes, dans laquelle le milieu de stockage comprend en outre un substitut de sérum sans sérum ou un agent anti-apoptotique.

11. Allogreffe selon la revendication 4, destinée à être utilisée dans un procédé transplantation allogénique, le procédé comprenant :
l'excision de tissu endommagé à partir du tissu receveur pour former un site receveur dans le tissu receveur ; et
l'insertion du tissu d'allogreffe excisé dans le site receveur.

12. Allogreffe destinée à être utilisée selon la revendication 11, dans laquelle le procédé comprend en outre :
l'alignement de la partie osseuse et la partie cartilagineuse du tissu d'allogreffe avec le tissu correspondant au niveau du site receveur.

13. Allogreffe destinée à être utilisée selon l'une quelconque des revendications 11 ou 12, dans laquelle le site receveur comprend un alésage cylindrique, et dans laquelle le procédé comprend en outre la mise en forme du tissu d'allogreffe excisé en une forme cylindrique.

14. Allogreffe destinée à être utilisée selon l'une quelconque des revendications 12 à 14, dans laquelle le procédé comprend en outre :
la mesure d'une profondeur du site receveur ; et
la découpe du tissu d'allogreffe à une longueur correspondant à la profondeur mesurée du site receveur.
